# EUROPEAN PATENT APPLICATION

(11) **EP 4 477 293 A1**
(43) Date of publication of application: **18.12.2024**
(21) Application number: 23770528.0
(22) Date of filing: 07.03.2023
(51) Int. Cl.: B01D 53/14, B01D 53/62, B01D 53/78, B01D 53/96, C01B 32/50, C07C 29/80, C07C 31/04

(54) **CARBON DIOXIDE RECOVERY SYSTEM**

(30) Priority: 16.03.2022 JP 2022040864
(71) Applicant: Mitsubishi Heavy Industries, Ltd., Tokyo 100-8332 (JP)
(72) Inventor: TACHIBANA, Shinya, Yokohama-shi, Kanagawa 220-8401 (JP); KATSUME, Tadashi, Yokohama-shi, Kanagawa 220-8401 (JP); YOSHIDA, Kaori, Yokohama-shi, Kanagawa 220-8401 (JP); YONEKAWA, Takahito, Yokohama-shi, Kanagawa 220-8401 (JP); NAGAOKA, Masaru, Takasago-shi, Hyogo 676-8686 (JP)
(74) Representative: Studio Torta S.p.A.
(86) International application number: PCT/JP2023/008481
(87) International publication number: WO 2023/176572

(57) **Abstract**

This carbon dioxide recovery system comprises: a first distillation column in which an absorption liquid that has absorbed carbon dioxide is heated so as to let carbon dioxide diffuse therefrom; a first reboiler which performs heat transfer between vapor and the absorption liquid extracted from the first distillation column; and a hydrogen feed unit which feeds hydrogen to the absorption liquid in the first reboiler, or to the absorption liquid that has flown out of the first reboiler.

## Description

### Technical Field

The present disclosure relates to a carbon dioxide recovery system.

The present application claims priority based on Japanese Patent Application No. 2022-040864 filed in Japan on March 16, 2022, the contents of which are incorporated herein by reference.

### Background Art

PTL 1 discloses a carbon dioxide recovery system including an absorption tower in which a gas containing carbon dioxide and an absorbing liquid are brought into gas-liquid contact with each other to allow the absorbing liquid to absorb the carbon dioxide, and a distillation tower that releases the carbon dioxide from the absorbing liquid that has absorbed the carbon dioxide in the absorption tower. In the carbon dioxide recovery system, a gas that is hardly dissolved in the absorbing liquid, for example, hydrogen is supplied to the distillation tower to promote the separation of the carbon dioxide from the absorbing liquid.

### Citation List

### Patent Literature

[PTL 1] Japanese Patent No. 6906766

### Summary of Invention

### Technical Problem

Normally, the distillation tower is designed such that the absorbing liquid in which the carbon dioxide is absorbed and the water vapor evaporated from the absorbing liquid in the distillation tower efficiently come into gas-liquid contact with each other. However, as in the carbon dioxide recovery system disclosed in PTL 1, when the hydrogen is supplied into the distillation tower from a lower end portion of the distillation tower, the water vapor and the hydrogen are not sufficiently mixed with each other. Therefore, there is a problem in that the absorbing liquid and the hydrogen cannot efficiently come into gas-liquid contact with each other. In addition, in each of a case where the hydrogen is supplied to the distillation tower and a case where the hydrogen is not supplied to the distillation tower, when the pressure in the distillation tower is set to the same condition, the amount of the water vapor moving from the absorbing liquid to the dry hydrogen is larger in the former case than in the latter case, and the reboiler duty is increased by the amount of the latent heat of the water vapor. Therefore, there is also a problem in that the water vapor is easily condensed.

In view of the above circumstances, an object of at least one embodiment of the present disclosure is to provide a carbon dioxide recovery system in which an absorbing liquid that has absorbed carbon dioxide and hydrogen can efficiently come into gas-liquid contact with each other in a distillation tower and a possibility that water vapor evaporated from the absorbing liquid is condensed can be reduced.

### Solution to Problem

In order to achieve the above object, a carbon dioxide recovery system according to the present disclosure includes a first distillation tower that heats an absorbing liquid in which carbon dioxide is absorbed to release the carbon dioxide from the absorbing liquid; a first reboiler that exchanges heat between the absorbing liquid extracted from the first distillation tower and steam; and a hydrogen supply unit that supplies hydrogen to the absorbing liquid in the first reboiler or the absorbing liquid flowing out from the first reboiler.

### Advantageous Effects of Invention

According to the carbon dioxide recovery system of the present disclosure, the hydrogen is supplied to the absorbing liquid in the first reboiler or the absorbing liquid flowing out from the first reboiler. In this manner, the hydrogen and the absorbing liquid are returned to the first distillation tower in a state of being mixed with each other. In this manner, the gas in which the hydrogen and the water vapor evaporated from the absorbing liquid are well mixed with each other rises inside the first distillation tower. Therefore, the absorbing liquid that has absorbed the carbon dioxide can efficiently come into gas-liquid contact with not only the water vapor but also the hydrogen. In addition, since the hydrogen is supplied to the absorbing liquid being warmed up in the first reboiler or the absorbing liquid warmed up in the first reboiler, the temperature of the first reboiler is controlled, and thus the probability of condensation of the water vapor can be reduced.

### Brief Description of Drawings

Fig. 1 is a schematic configuration diagram of a carbon dioxide recovery system according to Embodiment 1 of the present disclosure.
Fig. 2 is a schematic configuration diagram of a carbon dioxide recovery system according to Embodiment 2 of the present disclosure.
Fig. 3 is a schematic configuration diagram of a carbon dioxide recovery system according to Embodiment 3 of the present disclosure.
Fig. 4 is a schematic configuration diagram of a carbon dioxide recovery system according to Embodiment 4 of the present disclosure.

### Description of Embodiments

Hereinafter, a carbon dioxide recovery system according to embodiments of the present disclosure will be described with reference to the drawings. The embodiments which will be described below show aspects of the present disclosure and do not limit the disclosure, and any change can be made within the scope of the technical idea of the present disclosure.

### (Embodiment 1)

### <Configuration of Carbon Dioxide Recovery System According to Embodiment 1 of Present Disclosure>

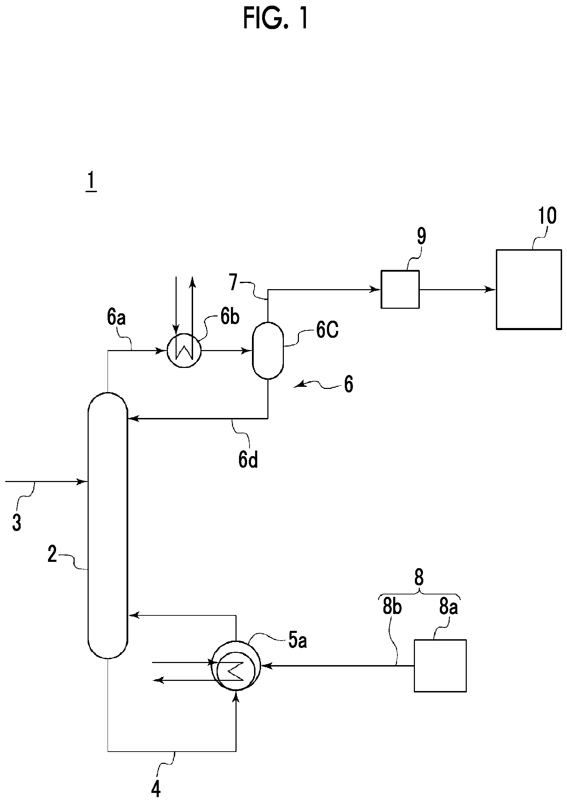

As shown in Fig. 1, a carbon dioxide recovery system 1 according to Embodiment 1 of the present disclosure includes a first distillation tower 2. The first distillation tower 2 communicates with, for example, an absorption tower (not shown) for absorbing carbon dioxide into an absorbing liquid by bringing a gas containing carbon dioxide into gas-liquid contact with the absorbing liquid via an absorbing liquid supply line 3.

The first distillation tower 2 is provided with a circulation line 4 for circulating the absorbing liquid such that the absorbing liquid in the first distillation tower 2 is extracted from the tower bottom of the first distillation tower 2 and returned to the first distillation tower 2 again. The circulation line 4 is provided with a first reboiler 5a. The first reboiler 5a is a heat exchanger that heats the absorbing liquid by exchanging heat between the absorbing liquid flowing through the circulation line 4 and the steam (for example, the water vapor).

The first reboiler 5a is provided with a hydrogen supply unit 8 that supplies hydrogen to the absorbing liquid in the first reboiler 5a. The configuration of the hydrogen supply unit 8 is not particularly limited. However, for example, the hydrogen supply unit 8 may include a hydrogen supply source 8a and a hydrogen supply line 8b in which one end communicates with the hydrogen supply source 8a and the other end communicates with a flow path for the absorbing liquid in the first reboiler 5a. The hydrogen supply source 8a may be, for example, a hydrogen cylinder or a hydrogen tank that stores hydrogen, or a hydrogen producing device that produces hydrogen by any method. A compressor may be provided in the hydrogen supply line 8b for supplying hydrogen from the hydrogen supply source 8a to the flow path for the absorbing liquid in the first reboiler 5a.

In addition, a heat exchanger may be provided in the hydrogen supply line 8b for supplying the hydrogen in a heated state.

The other end of the hydrogen supply line 8b may communicate with the circulation line 4 between the first reboiler 5a and the first distillation tower 2, instead of the flow path for the absorbing liquid in the first reboiler 5a. In this case, the hydrogen is supplied to the absorbing liquid flowing out from the first reboiler 5a.

In addition, the first distillation tower 2 is provided with a condensing device 6 that returns a condensed liquid obtained by cooling a gas (hereinafter, referred to as "outflow gas") flowing out from a tower top of the first distillation tower 2 to the first distillation tower 2. The condensing device 6 includes an outflow gas line 6a through which the outflow gas flows, a cooler 6b provided in the outflow gas line 6a, a recirculation tank 6c connected to a downstream-side end portion of the outflow gas line 6a, and a condensed liquid line 6d that returns the condensed liquid in the recirculation tank 6c to the first distillation tower 2. The cooler 6b can be, for example, a heat exchanger that cools the outflow gas by exchanging heat between the outflow gas and any cooling fluid. In addition to the condensed liquid, a gas mainly containing carbon dioxide and hydrogen as will be described later is present in the recirculation tank 6c. A gas supply line 7 that is connected to the device 10 that uses the gas may be connected to the tower top of the recirculation tank 6c. The gas supply line 7 may be provided with a compressor 9 for boosting the pressure of the gas.

### <Operation of Carbon Dioxide Recovery System According to Embodiment 1 of Present Disclosure>

Next, an operation of the carbon dioxide recovery system 1 according to Embodiment 1 of the present disclosure will be described. The absorbing liquid that has absorbed the carbon dioxide flows into the first distillation tower 2 via the absorbing liquid supply line 3. The absorbing liquid in the first distillation tower 2 is circulated to flow through the circulation line 4 after being extracted from the tower bottom of the first distillation tower 2, and then returns to the first distillation tower 2 again. When the absorbing liquid circulates in this way, the absorbing liquid flows inside the first reboiler 5a. When the absorbing liquid flows inside the first reboiler 5a, the hydrogen is supplied to the absorbing liquid by the hydrogen supply unit 8. In the first reboiler 5a, the mixed fluid of the hydrogen and the absorbing liquid is heated by exchanging the heat with the steam. The mixed fluid heated in the first reboiler 5a flows into the first distillation tower 2.

Since the temperature of the absorbing liquid staying in the first distillation tower 2 rises by the heated mixed fluid flowing into the first distillation tower 2, the carbon dioxide absorbed in the absorbing liquid is dissipated from the absorbing liquid and rises inside the first distillation tower 2. In addition, since water evaporates from the absorbing liquid, the water vapor also rises inside the first distillation tower 2. Further, the hydrogen in the mixed fluid heated in the first reboiler 5a is also dissipated from the absorbing liquid in the first distillation tower 2 and rises inside the first distillation tower 2. That is, the carbon dioxide, the water vapor, and the hydrogen rise in the first distillation tower 2.

The absorbing liquid flowing into the first distillation tower 2 via the absorbing liquid supply line 3 is heated by coming into gas-liquid contact with each of the carbon dioxide, the water vapor, and the hydrogen rising inside the first distillation tower 2 when falling in the first distillation tower 2. On the other hand, since the carbon dioxide, the water vapor, and the hydrogen are cooled, a portion of the water vapor is condensed into a liquid and falls in the first distillation tower 2. For this reason, the outflow gas flowing out from the tower top of the first distillation tower 2 contains carbon dioxide, water vapor, and hydrogen.

In Embodiment 1, since the hydrogen is supplied to the absorbing liquid in the first reboiler 5a, the hydrogen and the absorbing liquid flow into the first distillation tower 2 in a state where the hydrogen and the absorbing liquid are sufficiently mixed. In this manner, in the first distillation tower 2, the absorbing liquid is brought into gas-liquid contact with the water vapor and the hydrogen in a state where the water vapor and the hydrogen are sufficiently mixed with each other. Therefore, the absorbing liquid can efficiently come into gas-liquid contact with not only the water vapor but also the hydrogen. The same applies when the hydrogen is supplied to the absorbing liquid flowing out from the first reboiler 5a. In contrast, in a case where hydrogen is directly supplied into the first distillation tower 2, the water vapor and the hydrogen are not necessarily sufficiently mixed with each other, and a mixing device or the like may be required to sufficiently mix the water vapor and the hydrogen with each other. Therefore, the hydrogen supply method of Embodiment 1 is considered to be advantageous in the configuration of the carbon dioxide recovery system 1 from the viewpoint of bringing the absorbing liquid, the water vapor, and the hydrogen into gas-liquid contact with each other.

In addition, as described above, when the hydrogen is supplied to the first distillation tower 2, the water vapor is easily condensed. However, in Embodiment 1, the hydrogen is supplied to the absorbing liquid being warmed up in the first reboiler 5a or the absorbing liquid warmed up in the first reboiler 5a. Therefore, the temperature of the first reboiler 5a is controlled. In this manner, it is also possible to reduce a possibility that the water vapor is condensed.

The outflow gas flowing out from the tower top of the first distillation tower 2 flows through the outflow gas line 6a. When the outflow gas flows through the outflow gas line 6a, the outflow gas is cooled in the cooler 6b. When a temperature of the outflow gas is lowered by cooling, a component having a low boiling point in the outflow gas is condensed, but most of the carbon dioxide and the hydrogen remain in a gaseous state. When the cooled outflow gas flows into the recirculation tank 6c, the outflow gas is separated into a gas component and a liquid component. The gas component mainly containing carbon dioxide and hydrogen is supplied to the device 10 via the gas supply line 7. Meanwhile, the liquid component is returned into the first distillation tower 2 via the condensed liquid line 6d.

As described above, when the hydrogen is supplied to the first distillation tower 2, the reboiler duty of the first distillation tower 2 is increased. On the other hand, the temperature in the first distillation tower 2 can be lowered as compared with a case where the hydrogen is not supplied to the first distillation tower 2 by supplying the hydrogen to the first distillation tower 2. Therefore, the pressure in the first distillation tower 2 can be increased. In this case, the pressure in the recirculation tank 6c also increases. Therefore, in a case where the carbon dioxide and the hydrogen are supplied to the device 10 by the compressor 9, the energy consumption of the compressor 9 can be reduced. In addition, in this case, the pressure in the first distillation tower 2 is increased and the reboiler duty is reduced to the same level as when the hydrogen is not supplied. In this manner, it is possible to reduce the operating cost of the entire carbon dioxide recovery system 1.

### (Embodiment 2)

Next, a carbon dioxide recovery system according to Embodiment 2 will be described. The carbon dioxide recovery system according to Embodiment 2 is different from that of Embodiment 1 in that a second reboiler for heating the absorbing liquid is added in addition to the first reboiler 5a. Further, in Embodiment 2, the same components as those in Embodiment 1 are designated by the same reference signs, and the detailed descriptions thereof will not be repeated.

### <Configuration of Carbon Dioxide Recovery System According to Embodiment 2 of Present Disclosure>

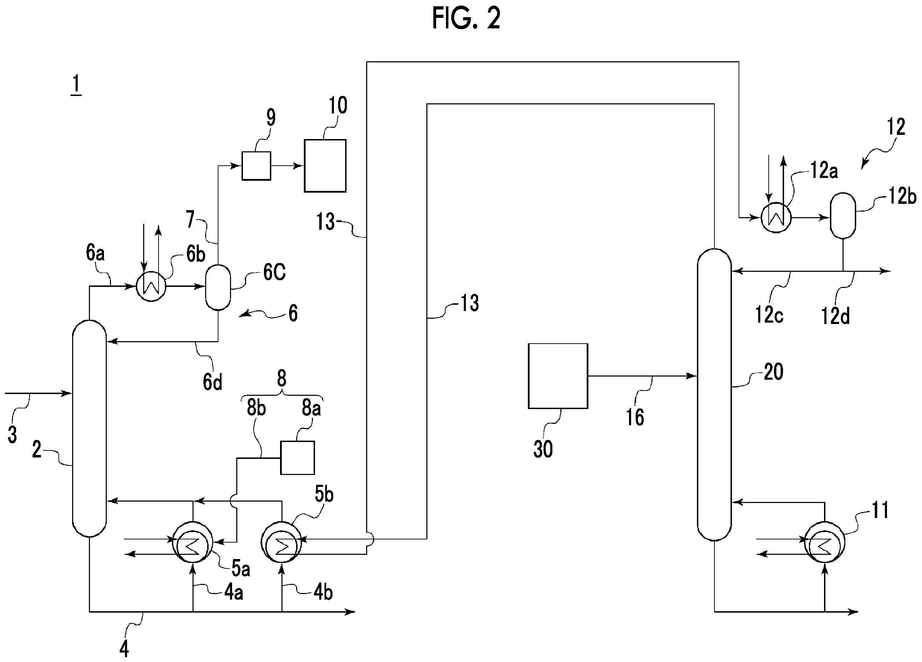

As shown in Fig. 2, a carbon dioxide recovery system 1 according to Embodiment 2 includes a second distillation tower 20 different from the first distillation tower 2 in addition to the first distillation tower 2. The circulation line 4 includes a first line portion 4a and a second line portion 4b, which are portions where the flow of the absorbing liquid is divided into two and flows in parallel to each other. The first reboiler 5a and the second reboiler 5b are provided in the first line portion 4a and the second line portion 4b, respectively. That is, the first reboiler 5a and the second reboiler 5b are provided in parallel with each other in a flow direction of the absorbing liquid. The second reboiler 5b is a heat exchanger that heats the absorbing liquid by exchanging heat between the absorbing liquid flowing through the second line portion 4b and the fluid supplied from the second distillation tower 20.

The second distillation tower 20 is not particularly limited as long as it is different from the first distillation tower 2 in configuration. However, in Embodiment 2, the second distillation tower 20 will be described as a distillation tower for refining crude methanol having methanol as a main component in a methanol production plant to obtain a fluid in which the methanol concentration is increased. The second distillation tower 20 communicates with the methanol production device 30 for producing the crude methanol via the crude methanol supply line 16.

As in the first distillation tower 2, the second distillation tower 20 is provided with the reboiler 11 for heating the crude methanol in the second distillation tower 20 and the condensing device 12 for condensing the methanol steam flowing out from the tower top of the second distillation tower 20. The reboiler 11 is, for example, a heat exchanger in which steam (for example, water vapor) and crude methanol exchange heat with each other. The condensing device 12 includes, for example, a cooler 12a that is a heat exchanger in which the methanol steam flowing out from the second distillation tower 20 and any cooling fluid exchange heat with each other, a recirculation tank 12b into which a fluid (mainly liquid methanol) cooled by the cooler 12a flows, a return line 12c for returning a part of the liquid methanol in the recirculation tank 12b to the second distillation tower 20, and a methanol supply line 12d for supplying the remaining liquid methanol in the recirculation tank 12b as a product.

One end of the line 13 through which the methanol steam flowing out from the second distillation tower 20 flows is connected to the tower top of the second distillation tower 20, and the other end of the line 13 is connected to the recirculation tank 12b of the condensing device 12. The line 13 is provided to pass through the second reboiler 5b, that is, to exchange heat between the absorbing liquid flowing through the second line portion 4b in the second reboiler 5b and the methanol steam flowing through the line 13. Therefore, in Embodiment 2, the fluid described above is methanol steam flowing out from the tower top of the second distillation tower 20. The other configurations are the same as those in Embodiment 1.

### <Operation of Carbon Dioxide Recovery System According to Embodiment 2 of Present Disclosure>

Next, an operation of the carbon dioxide recovery system 1 according to Embodiment 2 of the present disclosure will be described. The operation in which the carbon dioxide is dissipated from the absorbing liquid in the first distillation tower 2 and the operation in which the hydrogen is supplied to the absorbing liquid being warmed up in the first reboiler 5a or the absorbing liquid warmed up in the first reboiler 5a are the same as those in Embodiment 1. In Embodiment 2, the point in which the absorbing liquid extracted from the tower bottom of the first distillation tower 2 is heated not only in the first reboiler 5a but also in the second reboiler 5b is different from that in the Embodiment 1. Hereinafter, an operation different from that in Embodiment 1 will be described.

The crude methanol produced in the methanol production device 30 flows into the second distillation tower 20 via the crude methanol supply line 16. The crude methanol flowing into the second distillation tower 20 is heated in the reboiler 11, and the temperature thereof rises. When the temperature of the crude methanol rises, most of the methanol having a low boiling point is vaporized and rises inside the second distillation tower 20, while most of the components (mainly water) having a high boiling point remain in a liquid state in the second distillation tower 20. Steam (hereinafter, referred to as "methanol steam") having methanol flowing out from the second distillation tower 20 as a main component after rising inside the second distillation tower 20 flows through the line 13.

The methanol steam flowing through the line 13 exchanges heat with the absorbing liquid in the second reboiler 5b, and the temperature thereof is lowered. In some cases, at least a part of the methanol is condensed depending on the temperature. The methanol steam that has been heat-exchanged with the absorbing liquid in the second reboiler 5b flows through the line 13 in a state of the methanol steam or at least containing liquid methanol, and then flows into the recirculation tank 12b after being cooled by the cooler 12a in the condensing device 12. A part of the condensed liquid (liquid methanol) in the recirculation tank 12b is returned to the second distillation tower 20 via the return line 12c, and the remaining condensed liquid is supplied to a device (not shown) for consuming or storing methanol via the methanol supply line 12d.

In this way, the amount of steam used in the first reboiler 5a is reduced since the absorbing liquid is heated by exchanging heat between the steam and the absorbing liquid in the first reboiler 5a and the absorbing liquid is heated by exchanging heat between the fluid supplied from the second distillation tower 20 and the absorbing liquid in the second reboiler 5b. Therefore, the amount of heat supplied to the first distillation tower 2 from the outside can be reduced.

In Embodiment 2, at least a part of the methanol steam is condensed in the second reboiler 5b, so that the amount of heat equivalent to the latent heat of condensation can be given to the absorbing liquid. Accordingly, since the line 13 acts as a heat pump, the energy consumption of the entire carbon dioxide recovery system 1 can be reduced. In order to further enhance this effect, the inside of the second distillation tower 20 may be pressurized. For example, in order to pressurize the inside of the second distillation tower 20, a pump may be provided in the crude methanol supply line 16 to increase the pressure of the crude methanol supplied to the second distillation tower 20.

### (Embodiment 3)

Next, a carbon dioxide recovery system according to Embodiment 3 will be described. The carbon dioxide recovery system according to Embodiment 3 is different from that of Embodiment 2 in that a compressor that compresses the methanol steam supplied from the second distillation tower 20 to the second reboiler 5b is added to line 13. Further, in Embodiment 3, the same components as those in Embodiment 2 are designated by the same reference signs, and the detailed descriptions thereof will not be repeated.

### <Configuration of Carbon Dioxide Recovery System According to Embodiment 3 of Present Disclosure>

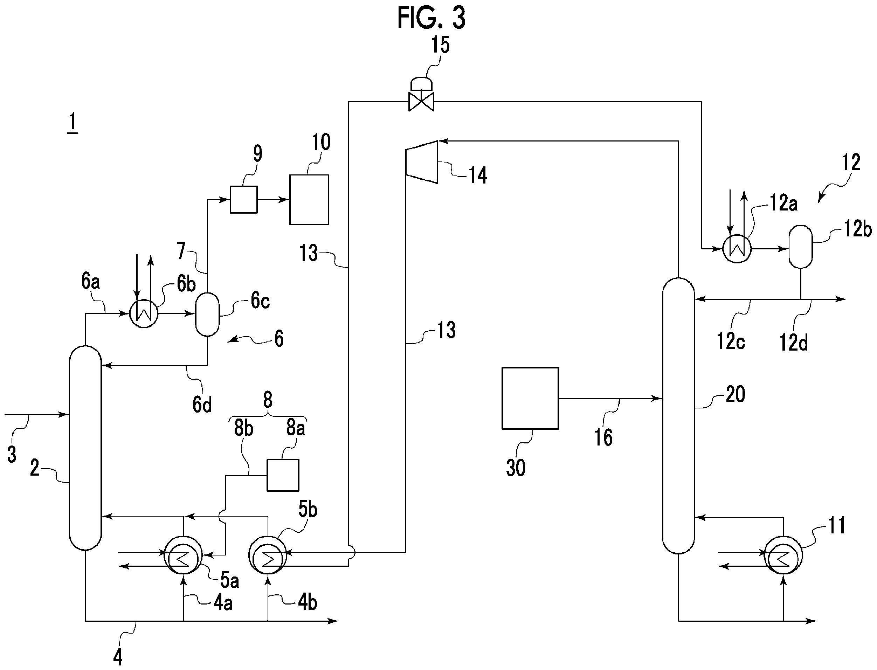

As shown in Fig. 3, in the carbon dioxide recovery system 1 according to Embodiment 3, the compressor 14 is provided between the second distillation tower 20 and the second reboiler 5b in the line 13, and the regulating valve 15 for regulating the pressure is provided between the second reboiler 5b and the condensing device 12. Other configurations are the same as those in Embodiment 2. The compressor 14 is for pressurizing the methanol steam supplied from the second distillation tower 20 toward the second reboiler 5b in place of pressurizing the inside of the second distillation tower 20 in Embodiment 2.

### <Operation of Carbon Dioxide Recovery System According to Embodiment 3 of Present Disclosure>

Next, an operation of the carbon dioxide recovery system 1 according to Embodiment 3 of the present disclosure will be described. Embodiment 3 is different from Embodiment 2 in an operation in which the absorbing liquid and the methanol steam exchange heat with each other in the second reboiler 5b. Hereinafter, only an operation different from that in Embodiment 2 will be described.

The methanol steam flowing out from the tower top of the second distillation tower 20 and flowing through the line 13 is compressed by the compressor 14. The temperature of the methanol steam is raised by the compression by the compressor 14. The methanol steam compressed by the compressor 14 exchanges heat with the absorbing liquid in the second reboiler 5b, and the temperature thereof is lowered. In some cases, at least a part of the methanol is condensed depending on the temperature. The methanol steam that has been heat-exchanged with the absorbing liquid in the second reboiler 5b flows through the line 13 in a state of the methanol steam or at least containing liquid methanol, and then flows into the recirculation tank 12b after being cooled by the cooler 12a in the condensing device 12. A part of the condensed liquid (liquid methanol) in the recirculation tank 12b is returned to the second distillation tower 20 via the return line 12c, and the remaining condensed liquid is supplied to a device (not shown) for consuming or storing methanol via the methanol supply line 12d.

In a case where the methanol steam flowing through the line 13 is used as the heat medium, the heat medium is compressed to raise the temperature thereof, and the amount of heat equivalent to the latent heat of condensation can also be given to the absorbing liquid by exchanging heat in the second reboiler 5b. Accordingly, the line 13 and the device associated with the line 13 act as a heat pump, and thus the energy consumption of the entire carbon dioxide recovery system 1 can be reduced.

In this way, the amount of steam used in the first reboiler 5a is reduced since the absorbing liquid is heated by exchanging heat between the steam and the absorbing liquid in the first reboiler 5a and the absorbing liquid is heated by exchanging heat between the absorbing liquid and the fluid supplied from the second distillation tower 20 after the fluid is compressed, in the second reboiler 5b. Therefore, the amount of heat supplied to the first distillation tower 2 from the outside can be reduced.

### [Embodiment 4]

Next, a carbon dioxide recovery system according to Embodiment 4 will be described. The carbon dioxide recovery system according to Embodiment 4 is different from that of Embodiment 2 or Embodiment 3 in that a modification in which the heat of the crude methanol produced in the methanol production device 30 is used in the second reboiler 5b is performed. In the following description, Embodiment 4 is configured in a form in which the above-described changes are made to Embodiment 3, but Embodiment 4 may be configured in a form in which the above-described changes are made to Embodiment 2. Further, in Embodiment 4, the same components as those in Embodiment 3 are designated by the same reference signs, and the detailed descriptions thereof will not be repeated.

### <Configuration of Carbon Dioxide Recovery System According to Embodiment 4 of Present Disclosure>

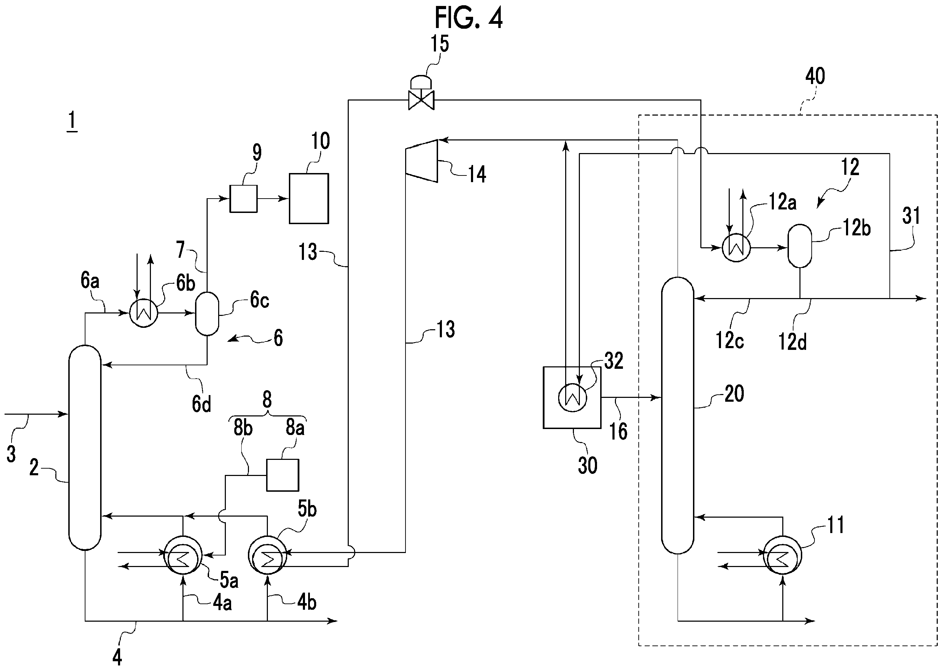

As shown in Fig. 4, in the carbon dioxide recovery system 1 according to Embodiment 4, the heat exchanger 32 for removing the heat generated during the methanol synthesis is provided in the methanol production device 30. The heat exchanger 32 is configured to exchange heat between the methanol supplied from the methanol purification device 40 configured to include the condensing device 12 and the second distillation tower 20 (including the reboiler 11) and the heat generated during the methanol synthesis. Although there is no particular limitation on where the methanol is supplied from the methanol purification device 40, in Embodiment 4, as a specific example, a configuration will be described in which a branch line 31 branched from the methanol supply line 12d is connected to the line 13 between the second distillation tower 20 and the compressor 14 so as to pass through the heat exchanger 32. The other configurations are the same as those in Embodiment 3.

### <Operation of Carbon Dioxide Recovery System According to Embodiment 4 of Present Disclosure>

Next, an operation of the carbon dioxide recovery system 1 according to Embodiment 4 of the present disclosure will be described. Embodiment 4 is different from Embodiment 3 in a part of the operation in which the absorbing liquid and the methanol steam exchange heat with each other in the second reboiler 5b. Hereinafter, only an operation different from that in Embodiment 3 will be described.

The crude methanol produced in the methanol production device 30 is cooled in the heat exchanger 32 by exchanging heat with the methanol supplied from the methanol purification device 40. On the other hand, the temperature of the methanol rises to become methanol steam. The methanol steam flows into the line 13 between the second distillation tower 20 and the compressor 14, and merges with the methanol steam flowing through the line 13 from the second distillation tower 20 toward the second reboiler 5b. The merged methanol steam is further warmed up by being compressed by the compressor 14, as in Embodiment 3, and exchanges heat with the absorbing liquid in the second reboiler 5b. Subsequent operations are the same as those in Embodiment 3.

In this way, the methanol steam heated in the heat exchanger 32 is supplied to the second reboiler 5b together with the methanol steam supplied from the second distillation tower 20. Accordingly, since the amount of the steam used in the first reboiler 5a can be further reduced, the amount of heat supplied to the first distillation tower 2 from the outside can be further reduced.

### <Modification Example of Carbon Dioxide Recovery System According to Each Embodiment>

In Embodiments 2 to 4, the second distillation tower 20 is a distillation tower for refining methanol in the methanol production plant. However, the present disclosure is not limited to the distillation tower, and may be a methanol-derived product producing device, for example, a device for producing dimethyl carbonate from carbon dioxide and methanol, a methanol-to-olefin (MTO) device, a methanol-to-gasoline (MTG) device, or the like. In addition, the methanol is not limited to a substance (a product or an intermediate material) produced in a plant, and may be used by using a substance externally procured as a raw material. In this case, for example, methanol is supplied from a storage facility of methanol and a heating device that heats methanol instead of the second distillation tower 20, which is a distillation tower. In addition, in Embodiments 2 to 4, the second distillation tower 20 is one distillation tower. However, the second distillation tower 20 may be configured by two or more distillation towers, or may be configured by a combination of one or more distillation towers and a device having a configuration other than the distillation tower.

In Embodiments 2 to 4, the fluid used as the heat source in the second reboiler 5b is the methanol steam, but the fluid is not limited to the methanol. Water or a hydrocarbon having a physical property that has a saturation temperature of 100°C or lower at an atmospheric pressure and 120°C or lower at 10 atmospheres or lower, or a hydrocarbon containing an oxygen atom may be used as the fluid. In addition, the substance is not limited to a pure substance, and a substance containing a part of the substance may be used as the fluid. As such a substance, ethanol, acetone, 2-propanol, hexane, a mixture thereof, or the like can be exemplified.

In Embodiments 2 to 4, the first reboiler 5a and the second reboiler 5b are provided in parallel with each other regarding a flow direction of the absorbing liquid, but the present disclosure is not limited to such forms. The first reboiler 5a and the second reboiler 5b may be provided in series with each other.

Although the configuration of the device 10 using mainly the gas containing the carbon dioxide and the hydrogen is not particularly mentioned in the present disclosure, the device using such a gas is not particularly limited. In the case of Embodiments 2 to 4, the device 10 may be the methanol production device 30.

The contents described in each embodiment are understood as follows, for example.

[1] A carbon dioxide recovery system according to one aspect includes a first distillation tower (2) that heats an absorbing liquid in which carbon dioxide is absorbed to release the carbon dioxide from the absorbing liquid; a first reboiler (Sa) that exchanges heat between the absorbing liquid extracted from the first distillation tower (2) and steam; and a hydrogen supply unit (8) that supplies hydrogen to the absorbing liquid in the first reboiler (5a) or the absorbing liquid flowing out from the first reboiler (Sa).

According to the carbon dioxide recovery system of the present disclosure, the hydrogen is supplied to the absorbing liquid in the first reboiler or the absorbing liquid flowing out from the first reboiler. In this manner, the hydrogen and the absorbing liquid are returned to the first distillation tower in a state of being mixed with each other. In this manner, the gas in which the hydrogen and the water vapor evaporated from the absorbing liquid are well mixed with each other rises inside the first distillation tower. Therefore, the absorbing liquid that has absorbed the carbon dioxide can efficiently come into gas-liquid contact with not only the water vapor but also the hydrogen. In addition, since the hydrogen is supplied to the absorbing liquid being warmed up in the first reboiler or the absorbing liquid warmed up in the first reboiler, the temperature of the first reboiler is controlled, and thus the probability of condensation of the water vapor can be reduced.

[2] The carbon dioxide recovery system according to another aspect is the carbon dioxide recovery system of [1], and further includes a second distillation tower (20) different from the first distillation tower (2); and a second reboiler (5b) that exchanges heat between the absorbing liquid extracted from the first distillation tower (2) and a fluid supplied from the second distillation tower (20).

According to such a configuration, the amount of steam used in the first reboiler is reduced since the absorbing liquid is heated by exchanging heat between the steam and the absorbing liquid in the first reboiler and the absorbing liquid is heated by exchanging heat between the fluid supplied from the second distillation tower and the absorbing liquid in the second reboiler. Therefore, the amount of heat supplied to the first distillation tower from the outside can be reduced.

[3] The carbon dioxide recovery system according to still another aspect is the carbon dioxide recovery system of [2], and further includes a compressor (14) that compresses the fluid before the fluid flows into the second reboiler (5b).

According to such a configuration, the amount of steam used in the first reboiler is reduced since the absorbing liquid is heated by exchanging heat between the steam and the absorbing liquid in the first reboiler and the absorbing liquid is heated by exchanging heat between the absorbing liquid and the fluid supplied from the second distillation tower after the fluid is compressed, in the second reboiler. Therefore, the amount of heat supplied to the first distillation tower from the outside can be reduced.

[4] The carbon dioxide recovery system according to still another aspect is the carbon dioxide recovery system of [3], in which the fluid contains water or a hydrocarbon having a physical property that has a saturation temperature of 100°C or lower at an atmospheric pressure and 120°C or lower at 10 atmospheres or lower, or a hydrocarbon containing an oxygen atom.

[5] The carbon dioxide recovery system according to still another aspect is the carbon dioxide recovery system according to any one of [2] to [4], and further includes a methanol production device (30) that produces crude methanol to be supplied to the second distillation tower (20), in which the fluid is methanol steam.

According to such a configuration, the amount of steam used in the first reboiler is reduced since the absorbing liquid is heated by exchanging heat between the steam and the absorbing liquid in the first reboiler and the absorbing liquid is heated by exchanging heat between the methanol steam supplied from the second distillation tower and the absorbing liquid in the second reboiler. Therefore, the amount of heat supplied to the first distillation tower from the outside can be reduced.

[6] The carbon dioxide recovery system according to still another aspect is the carbon dioxide recovery system of [5], and further includes a heat exchanger (32) that exchanges heat between the crude methanol produced by the methanol production device (30) and methanol to be supplied from a methanol purification device (40) that includes the second distillation tower (20) and purifies the crude methanol, in which methanol steam generated by exchanging heat between the crude methanol and the methanol in the heat exchanger (32) is configured to be merged with the methanol steam supplied from the second distillation tower (20).

According to such a configuration, the methanol steam heated in the heat exchanger is supplied to the second reboiler together with the methanol steam supplied from the second distillation tower. Accordingly, since the amount of the steam used in the first reboiler is further reduced, the amount of heat supplied to the first distillation tower from the outside can be further reduced.

### Reference Signs List

1: Carbon dioxide recovery system
2: First distillation tower
Sa: First reboiler
5b: Second reboiler
8: Hydrogen supply unit
14: Compressor
20: Second distillation tower
30: Methanol production device
32: Heat exchanger
40: Methanol purification device

## Claims

1. A carbon dioxide recovery system comprising:
a first distillation tower that heats an absorbing liquid in which carbon dioxide is absorbed to release the carbon dioxide from the absorbing liquid;
a first reboiler that exchanges heat between the absorbing liquid extracted from the first distillation tower and steam; and
a hydrogen supply unit that supplies hydrogen to the absorbing liquid in the first reboiler or the absorbing liquid flowing out from the first reboiler.

2. The carbon dioxide recovery system according to Claim 1, further comprising:
a second distillation tower different from the first distillation tower; and
a second reboiler that exchanges heat between the absorbing liquid extracted from the first distillation tower and a fluid supplied from the second distillation tower.

3. The carbon dioxide recovery system according to Claim 2, further comprising:
a compressor that compresses the fluid before the fluid flows into the second reboiler.

4. The carbon dioxide recovery system according to Claim 3,
wherein the fluid contains water or a hydrocarbon having a physical property that has a saturation temperature of 100°C or lower at an atmospheric pressure and 120°C or lower at 10 atmospheres or lower, or a hydrocarbon containing an oxygen atom.

5. The carbon dioxide recovery system according to any one of Claims 2 to 4, further comprising:
a methanol production device that produces crude methanol to be supplied to the second distillation tower,
wherein the fluid is methanol steam.

6. The carbon dioxide recovery system according to Claim 5, further comprising:
a heat exchanger that exchanges heat between the crude methanol produced by the methanol production device and methanol to be supplied from a methanol purification device that includes the second distillation tower and purifies the crude methanol,
wherein methanol steam generated by exchanging heat between the crude methanol and the methanol in the heat exchanger is configured to be merged with the methanol steam supplied from the second distillation tower.
